(19) Europäisches Patentamt
European Patent Office
Office européen des brevets

(11) **EP 4 385 468 A1**

(12) **EUROPEAN PATENT APPLICATION**

(43) Date of publication:
**19.06.2024 Bulletin 2024/25**

(51) International Patent Classification (IPC):
***A61F 9/008*** *(2006.01)*     ***A61F 9/009*** *(2006.01)*

(21) Application number: **22213247.4**

(22) Date of filing: **13.12.2022**

(52) Cooperative Patent Classification (CPC):
**A61F 9/008;** A61F 2009/00844

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **OD-OS GmbH**
**14513 Teltow (DE)**

(72) Inventors:
• **Friede, Sebastian**
  **10781 Berlin (DE)**
• **Mundt, Arne**
  **14476 Potsdam (DE)**
• **Paulauskas, Gediminas**
  **12165 Berlin (DE)**

(74) Representative: **Pfenning, Meinig & Partner mbB**
**Patent- und Rechtsanwälte**
**Joachimsthaler Straße 10-12**
**10719 Berlin (DE)**

(54) **TEMPERATURE MEASURING DEVICE WITH A SPECIAL MOUNTING SCHEME OF A PRESSURE CONVERTER**

(57) The invention refers to the measurement of tissue temperature during the treatment of biological tissue, in particular tissue in the eye (5) of a patient, by a therapy laser (1) for introducing energy into the tissue by means of controlled signals of the therapy laser in order to heat the tissue to produce a tissue reaction. During the treatment, temperature measurement laser signals (dedicated laser signals) (16a, 16b) are emitted to generate opto-acoustic pulses. A measuring device is provided with a measuring unit (7) comprising a ring-shaped pressure converter (7a, 8a, 11, 12) which is arranged to detect the opto-acoustic pulses generated by the laser signals for determining the temperature and/or an optical absorption of the tissue. A special mounting scheme of the pressure converter is provided to generate a clear and manageable sensor characteristic which can easily be aligned with the characteristic of the laser pulses.

Fig. 23

## Description

The invention refers to the measurement of a control value, in particular the temperature, of biological tissue, for example during treatment of the tissue, in particular in the area of ophthalmology.

Optical treatments of the human retina have become the quasi- standard method for treating diseases like diabetic retinopathy, retinal hemorrhages and diffuse macular edema. For some diseases photocoagulation may be the right way of a retinal photo therapy. In other cases, a so-called sub-threshold treatment turned out to be better suited.

One big problem for the sub-threshold treatment method is to tune in the parameters of the light sources (therapy lasers) used for the treatment to fit a therapeutic window: The treatment shall not harm or disable the functionality of retinal tissues like in the case of the photocoagulation treatment, but an under-treatment shall be avoided. So, the need for an appropriate dosing arises. An additional problem is posed by the fact, that the retinal pigmentation varies for different positions at the retina and for different eyes of individuals, yielding different photo absorptive characteristics and therefore different treatment results even with the same treatment light source parameters. In order to achieve the desired effects of some of the treatment methods, temperatures of above 60° C can be reached. For the success of an ophthalmological treatment, i

Treatment dosing can primarily be achieved by controlling the power and pulse scheme of a therapy laser. At the same time, an appropriate control value, for example the tissue temperature, has to be measured to form a basis for the control of the therapy laser. The measurement of an appropriate control value as for example the tissue temperature or a related parameter, may be implemented by using the photo-acoustic effect: A short exciting signal in the form of a light pulse (for example of a duration of 0.2 $\mu$s - 5 $\mu$s or 50 $\mu$s-100 $\mu$s) heats a region of the retina within a very short time, way below the coagulation threshold of the retinal tissues.

As a reaction to the heating, a reversible expansion of the retinal tissues takes place, which induces a transient pressure wave travelling through the vitreous to the front-eye. This pressure wave can be detected by measuring the periodic displacement or deformation of the cornea. Since the pressure wave is a result of the heating, and the heating, among other parameters, depends on the absolute temperature of the tissue and for example on the absorption strength of the tissue for the laser light, the current temperature value or a related control value which comprises the temperature as well as the absorption strength, can be derived from the measured intensity of the pressure wave and the power of the therapy laser, which is necessary to achieve a target tissue temperature or keep the current tissue temperature can be calculated or achieved by a control mechanism.

The sensing of the opto-acoustic response or strength/intensity of the pressure wave is in many cases realized by means of piezo-ceramic transducers coupled to the cornea via a contact-lens. Using a piezo-electric transducer as a pressure converter for converting the corneal displacement or deformation to electrical signals can be seen as one of the standard methods for opto-acoustic sensing at the eye. However, different concepts for sensing the pressure wave transferred by a corneal displacement or deformation may be used.

In the German patent specification DE 10135944C2 and a corresponding US patent document US2003/0032949A1, respective ophthalmologic treatments are described as well as a method for temperature measurement, wherein the temperature measurement comprises the application of radiation pulses to the tissue and the detection and analysis of photo- acoustic signals which are the result of tissue expansions and/or contractions. The pressure signals that occur as the result of the photo-acoustic effect are detected by a pressure converter in a contact lens which is brought in contact with the eye of the patient.

Considering the background of the prior art, it is a goal of the current invention to detect opto-acoustic pressure waves on a patient's eye with higher signal to noise ratio. To this end, it should be possible to choose the temperature measurement device appropriately to fit to the characteristics of the laser, which is used for producing the opto- acoustic pulses and the pulse shape as well as other parameters of the pulses. This laser can be the therapy laser or a separate exciting laser used for the measurement of a control value, in particular the temperature measurement, which is different from the therapy laser.

The invention therefore refers to a measuring device for measuring a control value, in particular the temperature, of the tissue of an eye of a patient with an excitation source for pulse-like excitation of the tissue by means of a beam falling through the eye lens, in particular a laser beam or laser signal, with a measuring unit for placing on the patient's eye for the detection and/or measurement of pressure waves which are generated by opto-acoustic pulses in the tissue and transferred from the eye through a pressure wave transfer body of the measuring unit to a pressure converter of the measuring unit for measuring transient pressure signals, wherein the pressure converter has the shape of a ring which at least partially consists of a piezoelectric material with an outer circumferential side, an inner circumferential side, a first front side and a second front side and is mechanically fixedly connected to the pressure wave transfer body, wherein only one of the sides of the ring-shaped pressure converter (the ring), the outer circumferential side or the inner circumferential side or the first front side or the second front side is mechanically fixedly connected to the pressure wave transfer body, while the other sides are not mechanically coupled to the pressure wave transfer body.

It should be noted that in this text, the expression "ring" generally means "ring-shaped pressure converter"

Currently used piezoelectric sensors exhibit resonance frequencies depending on the geometrical dimensions and the material. The transducers have to be optimized for the light source properties of the laser which is used for exciting the pulses for temperature measurement, since the opto-acoustic response depends on the light-matter interaction between the incident light (slope of the rising and falling edge) and the retinal tissue. Finite element simulations show, how to tune the resonance frequency of the piezo- transducers which are used as pressure converters. The tuning may be achieved by varying the thickness of the piezo ceramic element of the transducer, the inner diameter and the mounting scheme of the piezo ceramic transducer ring as well as the piezo transducer material (e.g., different types of lead zirconium titanates, like PZT-5A or PZT-5H). This enables for a tuning of the electro-mechanical properties according to the used laser characteristics for dedicated laser signals which are used for the measurement of a control value, in particular the tissue temperature.

For dosimetry applications at the retina, the state of the art sensing technique for opto- acoustic pressure waves are piezo-ceramic components. These piezo transducers in the past have been selected heuristically by the experience of engineers and scientists and have been suited for short dosimetry pulses of 200 ns or less.

The new approach, introduced by the invention, may build a foundation for matching exciting laser sources and the piezo ceramic transducer elements in a systematic way. An advantage of the new approach is the flexibility in the choice of laser sources. A broader portfolio of lasers may be used, and the piezo ceramics may be tailored accordingly to the laser properties or the chosen pulse characteristics.

The invention allows for an easier alignment of the resonance frequency of a piezo transducer for opto-acoustic pressure wave detection at the eyes retina with the characteristics of the laser used for the excitement of opto- acoustic pulses. The alignment may be realized by tuning the geometrical shape of the piezo ceramic and the mounting scheme of the transducer. This can be achieved either through measurements or with the help of finite element methods.

The optimized alignment of the laser characteristics and the pressure converter is especially useful when low power (< 5W) lasers in the microsecond pulsing regime are used (e.g. 0,1 $\mu$s -2 $\mu$s or 10 $\mu$s - 300 $\mu$s).

In the past, the shape, size and mounting scheme (the way, how the pressure converter is mounted or connected to a pressure wave transfer body) has not been considered systematically and the whole setup has rather been optimized heuristically and/or experimentally.

One problem that arises by connecting a piezo pressure converter ring or "ring-shaped pressure converter" to a pressure wave transfer body is that, depending on the mechanical coupling, a large variety of vibration modes of the ring may be excited. This leads to a distribution of resonance frequencies of the ring-shaped pressure converter which is difficult to predict or calculate, and difficult to reproduce since resonance frequencies in many cases are very sensitive to the geometrical parameters of the ring. This makes it difficult to calibrate such a sensor for temperature measurement.

It has been found, however, that the situation is much better predictable and reproducible, if, according to the invention, the transducer ring/ring-shaped pressure converter is mechanically connected to the pressure wave transfer body only on one of its sides. In This case, the resonance frequencies of the ring are dependent on the diameter and thickness of the ring in a much more transparent and predictable/calculable way and hence, the geometry of the ring and the characteristics of the laser pulses may much better be aligned than with other connection schemes.

The measuring device according to the invention may in one implementation be realized by that at least one, two or three of the sides of the ring-shaped pressure converter which is/are not fixedly connected to the pressure wave transfer body, is/are facing a surface of the pressure wave transfer body directly or through a mechanical decoupling layer.

It may further be provided that the ring-shaped pressure converter is on at least three of its sides or completely surrounded by the material of the pressure wave transfer body.

By these implementations, it can be achieved that the ring/pressure converter is well protected on one or more sides by the pressure wave transfer body without being mechanically fixedly connected or coupled with more than one of its sides to the body. This can be provided by an appropriate way of production, wherein e.g., the ring may be embedded in a decoupling layer or installed in a ring-shaped opening or groove inside the pressure wave transfer body and wherein one of the sides of the ring is fixedly connected to the pressure wave transfer body e.g., by an adhesive, i.e. a cohesive or material connection or a shape-fitting, positive locking connection or a force- fitting connection, e.g., by clamping it into the pressure wave transfer body.

A further implementation of the measuring device may provide that the ring-shaped pressure converter has a rectangular cross section.

In this case, the vibration modes are well manageable, reproducible and easily calculable. The ring is further easy to manufacture with the desired measures.

The device may further be realized by that the ring-shaped pressure converter is rounded, in particular oval or circular.

This quality of the ring also serves to ensure that the vibration modes are well manageable, reproduceable and easily calculable.

It may further be provided that the ring has an oval or circular cross section. In this case, each of the four sides of the ring-shaped pressure converter which can be connected to the pressure wave transfer body, can comprise 20% to 30% of the circumference of the cross section of the ring.

Another implementation of the measuring device may provide that the ring-shaped pressure converter has at least one damping element which is damping the vibration of the ring-shaped pressure converter.

Such a damping element may be provided to shape the resonance frequency of the ring and, e.g., widen the resonance peak. By this measure, potential peaks of resonance frequencies of the ring are less sharp and it is easier to align to a frequency close to the top of the peak because the slope of the curve is less steep than without a damping element.

Such damping elements may also added after the manufacturing process in order to align the vibration behavior of the ring experimentally after mounting.

The invention may for example be implemented by that the ring-shaped pressure converter has an outer layer at least on one of his sides made of a material which is damping the vibration of the ring. This kind of damping may also be implemented by a partial embedding of the ring in a damping layer which is located in an opening of the pressure wave transfer body.

Another implementation of the invention may provide that the pressure wave transfer body has the shape of a hollow cylinder or a hollow body which is cylindrical symmetric and/or that the pressure wave transfer body at a front end which is in use facing the eye of the patient, is provided with a disposable contact element for establishing direct contact with the eye of the patient, which is separably connected to the pressure wave transfer body by a snap-in connection.

Usually, the measuring unit of the device must be cleaned thoroughly after and/or before the use with a patient. This process can be simplified, and the consequences of an operational error may be reduced by using disposable contact elements at the end of the measuring unit which is in use facing the eye and making contact with the eye of the patient. The implementation described here ensures that the disposable contact element can be easily snapped in and out. It may also be provided that the separable disposable contact element can be cleaned and re-used later. This makes it possible to clean several contact elements centrally with a well manageable diligence and re- use them.

The disposable contact elements can be made of plastic or any other material that is appropriate for the transfer of pressure waves.

In case a disposable contact element is provided, this may mean that the pressure waves that are generated in the tissue of the eye, are transferred through the eye and from there to the disposable contact element and further to the pressure wave transfer body. If no disposable contact element is provided, the pressure waves may be directly transferred from the eye to the pressure wave transfer body or from the eye first to a part of a housing of the measuring unit and from there to the pressure wave transfer body and further to the pressure converter.

Another implementation of the invention may provide a receiving unit for the reception and evaluation of electrical signals which are generated by the pressure waves in the ring-shaped pressure converter. The electric signals reflect the signals of the pressure waves and can be used to calculate the strength /intensity of the pressure waves. A signal pre-processing unit may be provided to pre-process the electric signals from the pressure converter before sending them, either via a wire or wireless to the receiving unit.

The measuring unit can be implemented as a mobile measuring unit for a measuring device which apart from the pressure converter comprises a signal preprocessing unit, a data transmitting unit for wireless data transmission and an antenna.

In this case, the signal preprocessing unit is connected to the pressure converter and may comprise at least one, two, three, four, five, six or seven of the following elements: an amplitude modulator or a frequency modulator for modulating the measured signal on a radio wave, a signal amplifier, a band pass filter, a high pass filter, a low pass filter, a unit for digitizing data or signals, a unit for integrating over signals, a unit for averaging over signals or data and also rechargeable energy storage means.

It may be provided, that in a mobile measuring unit one, two, three or more than three of the following elements are placed inside a tubular housing:

a signal preprocessing unit, a data transmitting unit, a device for overvoltage protection, a device for short-circuit protection, a device for protection against incorrect polarity, a device for converting a first voltage level into a second voltage level, and a rechargeable energy storage means.

Further, an antenna for data transmission could be provided inside the tubular housing or on the outside of the tubular housing of the mobile measuring unit or in an opening of the tubular housing or an antenna can be formed by an opening of the tubular housing, wherein the antenna is connected to the data transmitting unit.

A potential implementation of the invention may further refer to a method for operating a measuring device of the kind described above comprising:

aligning the pulse-like excitation signals of the excitation source and the shape and size of the ring-shaped pressure converter and its connection to the pressure wave transfer body in such a way that the distribution of the frequencies of the opto-acoustic pressure waves has an initial shift relative to the distribution they would have to achieve the optimized resonance with the ring-shaped pressure converter or with the calculated mean mode frequency of the first 30 vibration modes of the ring.

The initial shift of frequency distribution could e.g., be more than 1%, more than 2% or more than 5 % of the frequency. By the initial shift, it can be ensured that a further, small and uncontrolled shift of the frequency distribution will lead to a predictable result because the relation between the excitation frequency and the excitation of the ring is clear, monotone, continuous and not too steep.

For further implementations of the invention and for more detailed features, the invention is shown in drawings and explained below.

Therein, the figures of the drawings are showing:

Figure 1:     a setup for ophthalmologic laser treatment with temperature monitoring according to the prior art,

Figure 2:     a setup for ophthalmologic laser treatment with a temperature measuring device including a mobile measuring unit,

Figure 3:     the internal structure of a mobile measuring unit,

Figure 4:     a structure of an antenna,

Figure 5:     a slit antenna in the housing of the mobile measuring unit,

Figure 6:     absorption spectra of different biological materials and laser emission wavelengths of different lasers,

Figure 7-12:  different patterns of therapy laser pulses and laser pulses intended for temperature measurement,

Figure 13:    a setup for laser treatment and a temperature measurement with a single laser and with a mobile measuring unit for the detection of pressure waves,

Figure 13a:   a part of a measuring unit with a disposable contact element,

Figure 13b:   a part of a measuring unit with another implementation of a disposable contact element,

Figure 14:    a cross section of a mobile measuring unit as schematically shown in Figure 13,

Figure 15:    a view inside the mobile measuring unit as indicated by the dotted line in Figure 14,

Figure 16:    the internal structure of a charging station for a mobile measuring unit,

Figure 17:    the internal structure of a mobile measuring unit,

Figure 18:    a perspective view of a pressure converter in the form of a piezo ring which, on its bottom side, shall be connected to a pressure wave transfer body,

Figure 19:    schematically the pressure converter of figure 18 and the way it is fixed/connected to a pressure wave transfer body,

Figure 20:    the relation between the mean eigenfrequency of the ring and the inner diameter of the ring as shown in figure 18 in case its bottom side is connected to a pressure wave transfer body, in a way as shown in fig. 19,

Figure 21:    the relation between the mean eigenfrequency of the ring and the thickness of the ring as shown in figure 18 in its axial direction in case its bottom side is connected to a pressure wave transfer body, in a way as shown in fig. 19,

Figure 22:    a perspective view of a pressure converter in the form of a piezo ring,

Figure 23:    schematically the pressure converter ring of figure 22 and the way its rim/outer circumference is fixed/connected to a pressure wave transfer body,

Figure 24:    the relation between the mean eigenfrequency of the ring and the thickness of the ring as shown in figure 22 in its axial direction in case its rim/outer circumference side is connected to a pressure wave transfer body, as shown in fig. 23,

Figure 25:    a cross sectional view of a pressure wave transfer body with a pressure converter,

Figure 26:    a cross sectional view of another pressure wave transfer body with a pressure converter and

Figure 27:    a cross sectional view of a pressure converter in the form of a toroid.

In many areas of ophthalmology, different energy sources, particularly lasers, are used for diagnostics and treatment. As a rule, the entire beamed-in energy is absorbed by the biological tissue and converted to heat, the resulting temperature increase achieving the desired treatment effect. For example, during laser photocoagulation, the retina of the eye is thermally coagulated in a targeted manner. In the case of the conventional irradiations with irradiation times about 100 ms, temperatures of above 60° C occur. Also, in the case of transpupillary thermotherapy (TTT), temperature increases are utilized for achieving a vascular occlusion. In the case of photodynamic therapy (PDT), a previously injected dye is activated by laser irradiation of a therapy laser on the ocular fundus. The active ingredient develops its effect only on those cells to which it is bound. In this case also, almost the entire beamed-in energy is absorbed in the dye and in the retina and is converted to heat. During the respective irradiation time (pulse duration) with relatively long treatment and radiation pulses in the order of from $\mu$s to several hundred seconds, a temperature increase of the treated biological tissue, particularly of the fundus of the eye, which results in unintended damage to regions of the retina, should in any case be avoided. A non-invasive real-time temperature determination during ophthalmologic laser treatment has been desired for a long time. As a solution, a technology has been developed which allows to monitor the tissue temperature during the thermal treatment of biological tissue, in particular during ophthalmologic treatments, by means of optoacoustic techniques. In more detail, it is known to determine the tissue temperature of biological tissue caused by a laser treatment with optoacoustic techniques by pulsed laser irradiation. Temperatures are measured at the ocular fundus for example during the selective microphoto coagulation or during any other treatment of diseases of the retina which uses an irradiation source like, for example, a therapy laser. A special laser pulse which is intended for temperature measurement generates a thermal tissue expansion or, at the end of the pulse, a thermal tissue contraction, wherein the expansion as well as the contraction generate pressure waves and wherein the amplitude or other characteristics of the pressure waves can be used for calibrating the pressure wave characteristics, e.g., the amplitude, to the temperature or to a more complex control value with a defined relation to the temperature and to an absorption characteristic of the ocular fundus. From the change of subsequent pressure wave characteristics after subsequent treatment pulses, the temperature increase or decrease and, in addition, the respective absolute temperatures can be determined by means of the relation between the temperature of the tissue and the pressure wave characteristics, in particular the pressure wave amplitude, which is defined by the Grüneisen coefficient. It is an object of the current invention to provide a simplified method and a system of the kind described above for the non-invasive determination of the control value mentioned above or of the temperature on treated biological tissue, which can also be an implementation of a control value. The control value or the measured temperature may then, as already explained above, serve for controlling the power of the therapy laser. The control value may for example be the current temperature of the treated tissue of the eye, which can be determined based on the measurement of the strength of the opto-acoustic pulses or the detected pressure waves. For example, the strength of the pressure waves, their amplitude, average amplitude or accumulated energy may be measured or determined.

The measured strength of the pressure waves depends on the tissue temperature, in which the pressure waves are generated, the absorption of the dedicated laser signals on their way to the tissue and the intensity of absorption of the laser signals in the tissue (hence, for example: the pigmentation strength). In the range of parameters, where the treatment generally takes place, a variation of the strength of the detected pressure waves dP is determined by the following equation:

$$dP = \mu\,\Gamma\,dH,$$

wherein dP is the Variation of the strength of the pressure waves, $\mu$ ist he absorption strenght of laser light in the tissue, $\Gamma$ is the Grüneisen coefficient and dH is the accumulated laser beam energy in the tissue (heat dissipation in the tissue has been neglected for short time ranges). Because both parameters, the tissue temperature and the absorption strength of the tissue, point into the same direction (high temperature and high absorption strength require less power of the therapy laser), the measured strength of the pressure waves is an appropriate control value for controlling the power of the therapy laser. Therein, it is one option to use the control value as such for the control of the therapy laser, but it is another option to first determine the current temperature of the tissue, for example based on a calibration, and after that to control the therapy laser based on the determined temperature value.

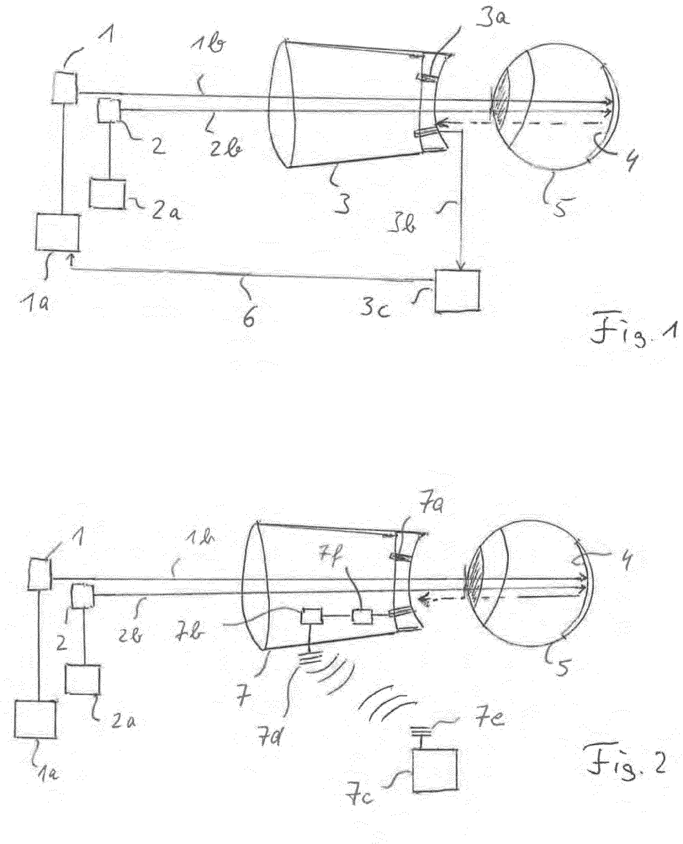

Figure 1 shows a setup with a therapy laser 1 which is controlled by a therapy laser control unit 1a and a measuring device which can be a temperature measuring device with an excitation source which is formed by an excitation laser 2 with a control unit 2a for the excitation laser 2 and a measuring unit 3 for measuring transient pressure waves.

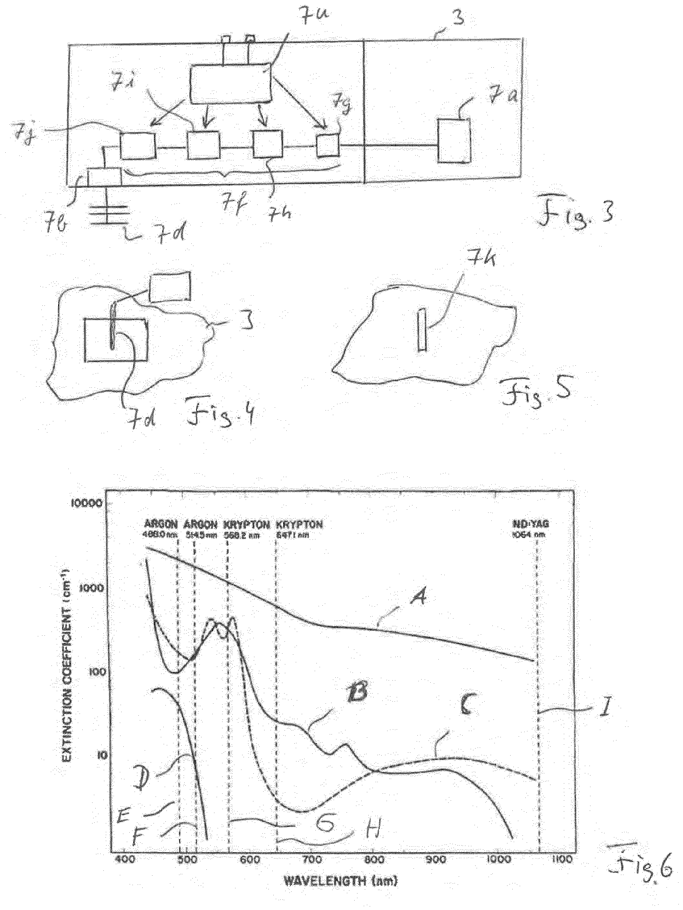

The therapy laser 1 generates a therapy laser beam 1b which is controlled to heat the biological tissue at the fundus 4 of the eye 5 of a patient. The characteristics of the therapy laser beam can be chosen appropriately dependent on the treatment that is needed. The therapy laser beam may have the form of a continuous wave or of repeated pulses or any other form and different laser types can be used according to the disease that shall be treated and the wavelength of the radiation that shall be provided and that is absorbed in the eye. An overview over some laser types that are available is given in Figure 6 and described below.

The excitation laser 2 is controlled to generate a laser beam 2b forming signals that generate thermic expansions and/or contractions in the or close to the target area of the therapy laser in the eye. The thermic expansions and/or contractions generate pressure waves that are sent out from the biological tissue through the eye 5 and can be detected by a measuring unit 3 for measuring transient pressure waves. The pressure waves can be detected by a transducer/pressure converter 3a and transformed in electrical signals, which are then sent through cables 3b to a signal processing unit 3c. The signal processing unit 3c can derive a current control value or, more specific, a temperature value from the signals that are detected by the transducer or pressure converter 3a. If the control value is not exactly the tissue temperature, it may be a value which is related to the tissue temperature and an absorption strength in the tissue of the laser light forming the dedicated laser signals of the eye. More in detail, a variation of the control value may directly be proportional to a variation of a product of the tissue temperature and the absorption strength in the tissue of the laser light forming the dedicated laser signals. The calculated temperature or the control value or its variation may for example, serve to control the therapy laser power appropriately. For this purpose, a feedback loop connection 6 can be provided between the signal processing unit 3c and the therapy laser control unit 1a.

The transient pressure wave signals that are measured by the transducer/pressure converter 3a are very high frequent signals. Therefore, the transmission of these signals over cables 3b is prone to a lot of disturbances creating errors in the determination of temperatures. This is even enhanced by any potential movement of the cables, which is difficult to avoid when the unit 3 and/or the patient is moving. Further, the cables can disturb the installation whenever the whole treatment device is set up for use.

Figure 2 therefore is showing a new setup with a mobile measuring unit 7 which comprises a data transmitting unit 7b for wireless data transmission and an antenna 7d. On the receiving side, a receiving unit 7c is provided separately from the mobile measuring unit 7. The receiving unit 7c is connected to an antenna 7e for the wireless reception of signals/data.

The wireless transmission of data, in particular of digitized or otherwise preprocessed data, is less prone to disturbances than the transmission by movable cables.

The reliability of the signal/data transmission can be enhanced by the preprocessing of the transient pressure wave signals on the side of the mobile measuring unit 7. For this purpose, a preprocessing unit 7f is provided.

A preprocessing unit 7f may comprise or be formed by a unit for modulating the measured signals on a radio wave, e.g. in the form of amplitude modulation or frequency modulation. The preprocessing unit may also comprise or be formed by an analog to digital converter and use a digital transmitter and a standardized protocol for digital data transfer like Bluetooth, Wifi, Zigbee, ethernet or any other standard.

The preprocessing unit may further comprise different elements, for example, a high pass filter and/or a low pass filter and/or a bandpass filter, an amplifier, an analog/digital converter, a unit for integrating a signal over time and an averaging unit that can determine average values from several results. A concrete example for the structure of a preprocessing unit will be shown in another figure and described below.

An antenna 7d for wireless communication can be formed in various ways. It can for example be a simple rod that is integrated in the wall of a tubular housing of the mobile measuring unit. The housing may be made of a nonconductive material and in this case, the antenna may simply be positioned inside the housing. If the housing is electrically conductive, the antenna shall be isolated from the housing. The antenna can protrude from the housing, or it can be printed as a conductive layer on the outside of the housing. If the housing is conductive, an isolating interlayer must be provided.

The antenna can also be provided in an opening of the housing, or it can be formed, in case the housing is made of a conductive material, as an opening, for example as a slit, which in this case must be coupled to the transmitter appropriately.

The transducer or pressure converter for sensing/detecting the pressure waves may also have one of several different forms which are in any form sensitive to pressure waves and change a physical parameter if they are hit by a pressure wave. Well known forms of such sensors are dielectric materials which change their dielectric constant or, in other words, their refractive index if they are hit by a pressure wave. The influence of the changing refractive index on light that passes the sensor can be measured. One of those sensors is for example an interferometer. Another kind of sensor for pressure waves are piezoelectric elements. The pressure converters 3a, 7a in figures 1 and 2 are just examples. A concrete implementation is described below.

Figure 3 shows an example of the structure of a preprocessing unit 7f which is coupled to a pressure converter 7a and a transmitter 7b. The mobile measuring unit 3 is represented schematically as a box. The preprocessing unit 7f is comprising a filter 7g, a signal integrator 7h, an averaging unit 7i and an analogue to digital converter 7j. From the pressure converter 7a, signals are sent to the filter7g, the filtered signals are integrated in the signal integrator 7h and after several laser pulses, an average is calculated over the integrated signals for all photo-acoustic pressure waves by the averaging unit 7i. In more detail, for each transient pressure wave signal, the signal is integrated and an average is calculated from the single integrated values. After digitization of the average value in the A/D converter 7j, the digital data are transmitted wirelessly over the antenna 7d by a transmitting unit 7b. A battery 7u serves as rechargeable energy storage means and may be connected with all elements of the mobile measuring unit which need energy supply.

In Figure 4, a rod shape antenna is shown which is positioned in an opening of the metallic housing 3. Figure 5 shows an antenna 7k in the form of a slit opening in a metallic housing.

In Figure 6, four different absorption curves (extinction coefficients) are shown for different materials between 400 and 1100 nanometer. Curve A represents melanin, curve B reduced hemoglobin, curve C: oxygenated hemoglobin and curve D: macular xanthophyll.

The five vertical lines show the specific emission wavelengths of different laser models: Line E is representing an Argon laser with a wavelength of 488 nm, line F an Argon laser at the wavelength of 514,5 nm, line G a Krypton laser at a wavelength of 568,2nm, line H a Krypton laser at a wavelength of 647,1 nm and line I a NDYAG laser at a wavelength of 1064 nm. The appropriate therapy laser must be chosen dependent on the intended therapy and the substance that shall absorb the laser light and hence dependent on the absorption curve of the substance.

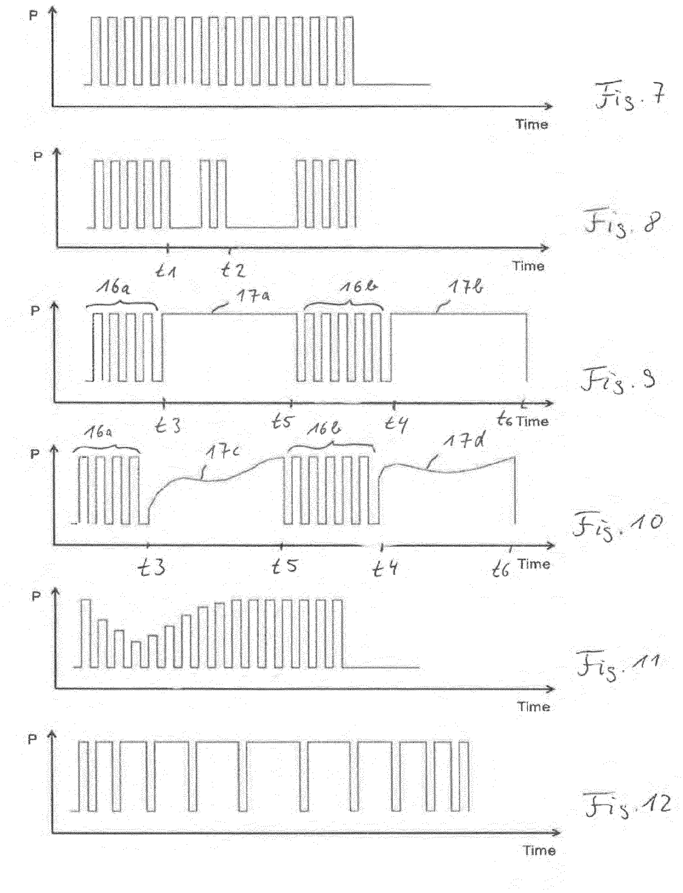

In figures 7-12, different patterns of laser activity of a therapy laser are shown over time, wherein the therapy laser delivers not only laser signals for therapeutic treatment, but also dedicated laser signals.

On the vertical axis, the laser intensity, e.g., the laser power is shown while the horizontal axis represents the running time. The diagrams are not calibrated. They shall just serve to explain the principles of the aligned laser activities wherein on one hand the therapy laser signals serve to heat biological tissue for a medical treatment and on the other hand the dedicated laser signals generate opto-acoustic laser pulses for the purpose of temperature measurement or for the purpose of determining a control value. The relation of the duration of the single pulses which shall do the medical treatment and the pulses for generation of opto-acoustic signals (dedicated laser signals) are just examples and shall in no way limit the scope of the claimed invention. The same holds for the representation of pulse pauses between the laser pulses, which are described to have a certain length even if the relation of pulses and pauses in the graphics may not be represented to scale.

Of course, the laser activity which is dedicated to the generation of optoacoustic pulses (dedicated laser signals) can also contribute to a heating of the tissue and if this contribution is considerable, it must be taken into account when the laser activity for the treatment as such is determined or controlled. The time intervals are described using the unit "µs", which shall mean throughout this text the same as the expression "microsecond".

The laser activities in figures 7 to 12 may represent laser pulses of both kinds, that is to say, laser pulses for medical treatment and laser pulses for the generation of opto-acoustic pressure waves for temperature measurement or for the determination of a control value which is not exactly the tissue temperature, wherein both kinds of pulses may be generated by one and the same laser (a therapy laser), but the graphics may also be interpreted as showing pulses from at least two different lasers, i.e. a therapy laser and a separate laser for the generation of dedicated laser signals, which are combined so that the laser beams of both or all lasers are directed to the same tissue. In this case, it would be an advantage to combine the control of all lasers that are used in combination.

Generally, the duration of both kinds of laser signals (dedicated laser signals and laser signals which only serve for the therapy) and the steepness of the flanks of the pulses can be controlled by a laser control unit electronically or by an optical switch. A mechanical chopper may also be used. If optical switches are used, it may be an advantage, if the laser beams pass a transparent body, like a glass or plastic body, e.g. an optical fiber, wherein a parameter of the material can be controlled to control and switch on and off the optical beam which passes the material. This way, an optical switch may be formed as is generally known from the prior art.

Figure 7 shows a continuous pulse train of micro-pulses with a duration of 1 µs - 2 µs, or 25 µs - 50 µs wherein, in both cases, at least 25 µs pulse pauses are provided between the individual pulses. Each pulse is used for an incremental heating and a selected number of pulses, for example a selected number of subsequent pulses, or every second or third or tenth pulse, may also be dedicated laser signals and hence, be used for the generation and detection of opto-acoustic transients/pressure wave signals. The measured pressure wave signals may be integrated and averaged (e.g. average of 5 to 25 or 5 to 50 measurements).

Generally, a laser pulse may be defined as a dedicated laser pulse or dedicated laser signal, if it is actually used to measure a control value or in particular the tissue temperature, that is, if the opto- acoustic pulse ore pressure wave transient generated by the laser pulse is actually detected and used for determining a control value as mentioned above or in particular the tissue temperature.

Figure 8 shows an uninterrupted pulse train of micropulses (0,1 µs - 2 µs, 1 - 2 µs or 25 µs - 50 µs) wherein, in both cases, at least 25 µs pulse pauses are provided between the individual pulses. Each pulse is used for an incremental heating and for the measurement of opto-acoustic transients. The measured pressure wave signals may be averaged (5 to 50 averages). It can also be decided that only every second or third or any other selection of the pulses is used for temperature measurement or for the determination of a control value. If a target temperature of the tissue or a target control vlue is reached, the duration of the pauses between the pulses may be increased, as shown at the times designated as t1 and t2, for letting cool down the tissue by heat diffusion. The pulse pauses may be decreased or the duration of the pulses or a selection of pulses or a series of pulses may be prolongated if the temperature or the control value falls below a threshold of the target control value or the target temperature for heating up the tissue.

Figure 9 shows continuous pulse trains 16a, 16b of micro-pulses (1 µs - 2 µs) with at least 25 µs pulse pause between the individual pulses that are used for the temperature measurement and which therefore are dedicated laser signals 16a, 16b. Heating pulses 17a, 17b of a longer duration (50 μs - 20 ms), starting at times designated as t3 and t4, may be applied between the measurement pulses (dedicated laser signals). After a heating period, shorter measurement pulses (dedicated laser signals) 16b, starting at t5 and t6, are applied for determining the applied dose. The pulse length of the following heating pulse may be altered in order to heat the tissue slightly or to a larger extent. The heating period may be skipped if it is necessary to let the tissue cool down.

Figure 10 shows a continuous pulse train of micro-pulses (1 μs - 2 μs) 16a with at least 25 μs pulse pauses between the pulses, which are used for the temperature determination (dedicated laser signals). It can also be decided that the pauses between the pulses may be shorter than 25 μs. Heating pulses 17c, 17d of a duration between 50 μs and 20 ms may be applied between the measurement pulses (dedicated laser signals) 16a, 16b. The power of the heating pulses may be modulated or adapted in order to heat the tissue to an extent which is appropriate to reach the target temperature of the tissue. After each heating period, shorter measurement pulses are applied for determining the tissue temperature. The duration of the heating pulses may be more than two times, more than five times or more than ten times the duration of the measurement pulses. Each heating period may be skipped for cooling the tissue if the tissue temperature is higher than the target temperature and/or the control value is higher than a target control value.

Figure 11 shows a continuous pulse train of micro-pulses (1 μs - 2 μs, or 25 μs - 50 μs) with at least 25 μs pulse pauses between the individual pulses. Each pulse is used for an incremental heating and can also be used for the measurement of opto-acoustic transients if the respective pressure wave signals are detected. The measured signal may be integrated for each single pressure wave/opto-acoustic pulse and averaged (average over 5 to 25 or 50 measurements). The power of the pulses may be controlled/adapted in the form of an amplitude modulation for a control of the heating. Smaller amplitudes of the laser intensity slow down the heating or may lead to a cooling down of the tissue.

This might be useful for heating the tissue close to treatment limit temperatures or for cooling the tissue slowly. For a faster cooling, a pulse sequence may be skipped.

Figure 12 shows a continuous pulse train of micro-pulses (1 μs - 2 μs, or 25 μs - 50 μs) with at least 25 μs pulse pause between the individual pulses. Each pulse is used for an incremental heating and/or for the measurement of optoacoustic transients. The measured signal may be averaged over 5 to 25 or 50 measurements. The duration of the pulses may be controlled/adapted for a control of the heating or the tissue temperature. Hence, the laser pulses are controlled in the form of a pulse width modulation/ frequency modulation.

A shorter duration of the laser pulses in relation to the pulse pauses slows down the heating. This might be useful for heating the tissue close to treatment limit temperatures or for cooling the tissue slowly. Larger pulse durations may be used to heat up the tissue quickly. For a fast cooling, the pulse sequence may be turned off for a certain time.

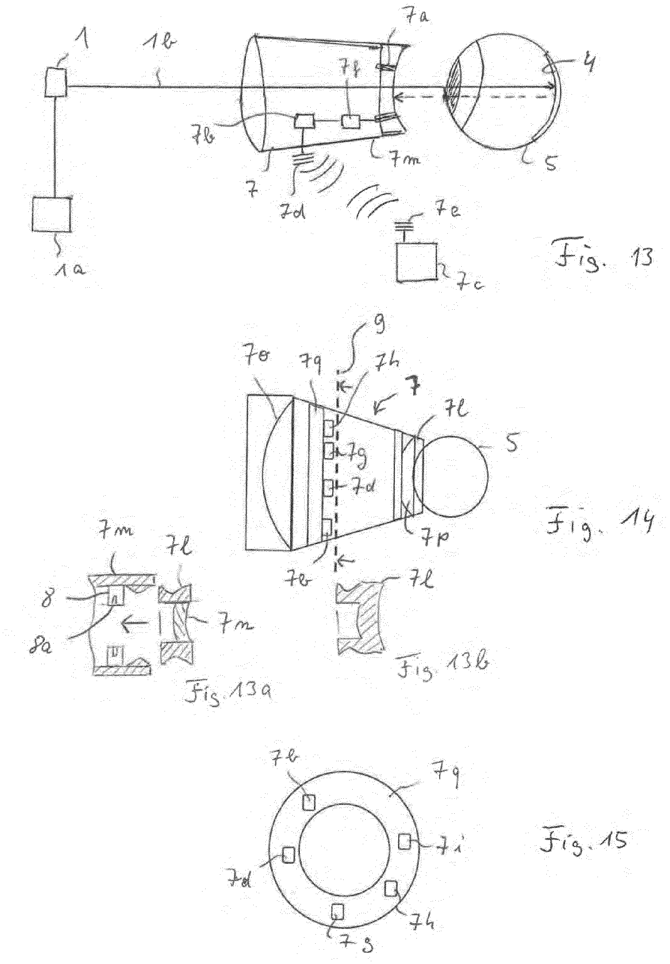

Figure 13 shows a setup of a laser treatment device with a measuring device for a control value, in particular a temperature measuring device, wherein the therapy laser 1 is emitting the laser beam 1b to the fundus 4 of the patient's eye and wherein the therapy laser is controlled by the control unit 1a in a way as to emit not only laser signals for the medical treatment and exclusively for purposes of heating the tissue at the fundus of the eye, but also dedicated laser signals which are used to detect pressure waves/transients which are generated by the dedicated laser signals. The detection of the pressure waves/transients happens in the same way as described with reference to figure 2 and using the same reference numbers for the same elements.

The control unit can be specially arranged to distinguish between laser signals which are intended just for heating and medical treatment of the tissue and dedicated laser signals that are intended to be used for determining a control value or specifically for temperature measurement. In the case that the control unit 1a provides a dedicated laser signal, it may send a signal to the temperature measuring device and align with this device, so that the (temperature) measuring device can use the dedicated signal and prepare for the measurement of the respective pressure wave/transient. The control unit may as well communicate with the measuring device/temperature measuring device about a time schedule of one, one to ten or one to fifty following dedicated laser signals. A data processing unit of the temperature measuring device may be combined with or integrated in the receiving unit 7c, so that the temperature or a control value may be finally calculated in this unit. A feedback loop 6 as shown in figure 1 between the receiving unit 7c and the control unit 1a may be provided to allow the control unit to control the therapy laser appropriately to reach and keep a target tissue temperature.

Figure 14 shows a cross section of a measuring unit for measuring pressure waves, which preferably may be implemented as a mobile unit 7. In the figure, the measuring unit is shown in mechanical contact with an eye 5. The mobile measuring unit 7 is pressed onto the eye with a certain pressure force for establishing good conditions to ensure that pressure waves are efficiently transported or transferred from the fundus through the eye body to the measuring unit and in the measuring unit to a pressure wave sensor/pressure converter.

One detail of the measuring unit 7 and its quality to ensure a good transmission or transfer of the pressure waves to the pressure wave sensor may be explained with reference to the figures 13a and 13b. Figure 13a shows an end part of the housing wall 7m of the measuring unit 7, wherein a pressure wave transfer body 8 is provided and fixed to the housing wall 7m.

The pressure wave transfer body 8 may also be formed by the housing wall 7 or be combined with it. The pressure wave transfer body 8 may contain or hold a pressure wave sensor/pressure converter in the form of any sensor which in this text is described as a potential implementation of such sensor. The Sensor may particularly be implemented as a piezo sensor 8a in the form of a ring of a piezo sensitive material which is fixedly mechanically connected to the pressure wave transfer body 8. The ring may as well only partially consist of a piezo sensitive material.

In figures 13a and 13b, a contact element 7l is shown which is provided for making direct contact to the patient's eye and transferring the pressure waves from the eye to the pressure wave transfer body 8. The measurement unit, which is used for different patients, either has to be cleaned and disinfected before use or the contact element can be implemented as a disposable contact element 7l or at least as an element which is separable from the housing of the measuring unit.

Figures 13a and 13b show a disposable contact element 7l which can be snapped in on the housing of the measuring unit 7 and has therefore a certain elasticity. The contact element 7l may be combined with a transparent cover 7n which covers the opening of the ring-shaped contact element, so that laser beams may pass through. In another implementation, the cover 7n and the contact element may be combined and made of the same transparent material as shown in figure 13b.

Figure 14 shows that the measuring unit 7 has a conical, tubular housing which at both ends is covered by a lens 7o, 7p. Instead of each of the lenses, on each side another optical element, like, e.g. flat glass or plastic, may be provided which is transparent at least for the laser beams used by the device. In one potential implementation, only one or no side of the tubular housing is covered by a transparent cover element.

In the tubular housing, at least one or two rings 7q may be provided and fixed to the inner side of the housing wall 7m. The ring 7q has an opening to let the laser beam pass through. On one or both front sides of the ring 7q, elements 7b, 7d, 7g,7g, 7h, 7i, 7j, 7k, of the preprocessing unit may be installed and the ring 7q itself may be implemented as a printed circuit board with printed or etched conductors.

Figure 15 is showing a front side view of the ring 7q as indicated by the dotted line 9 in figure 14.

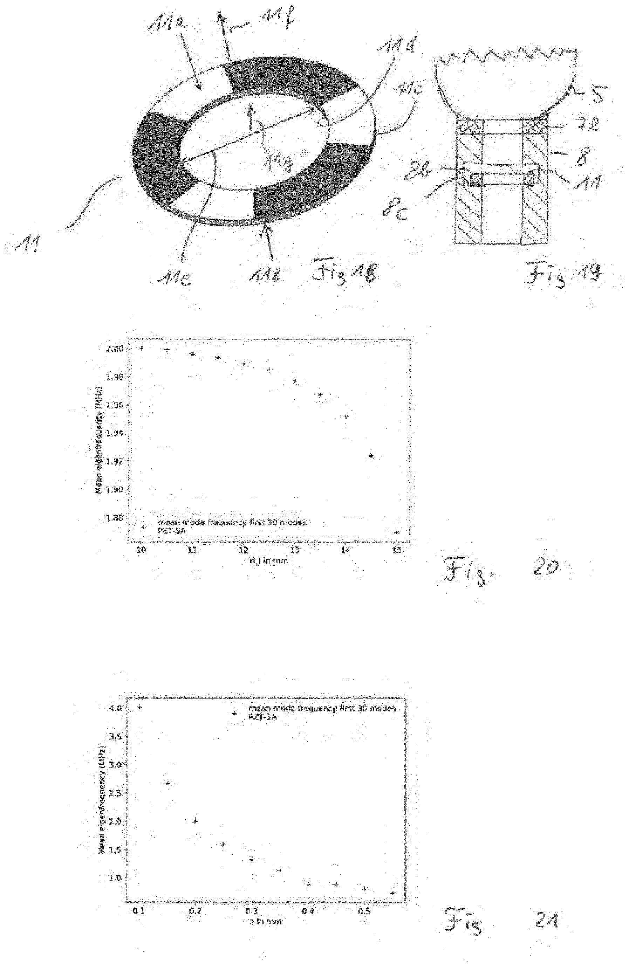

Further information about the pressure converter itself will be given with reference to figures 18ff below.

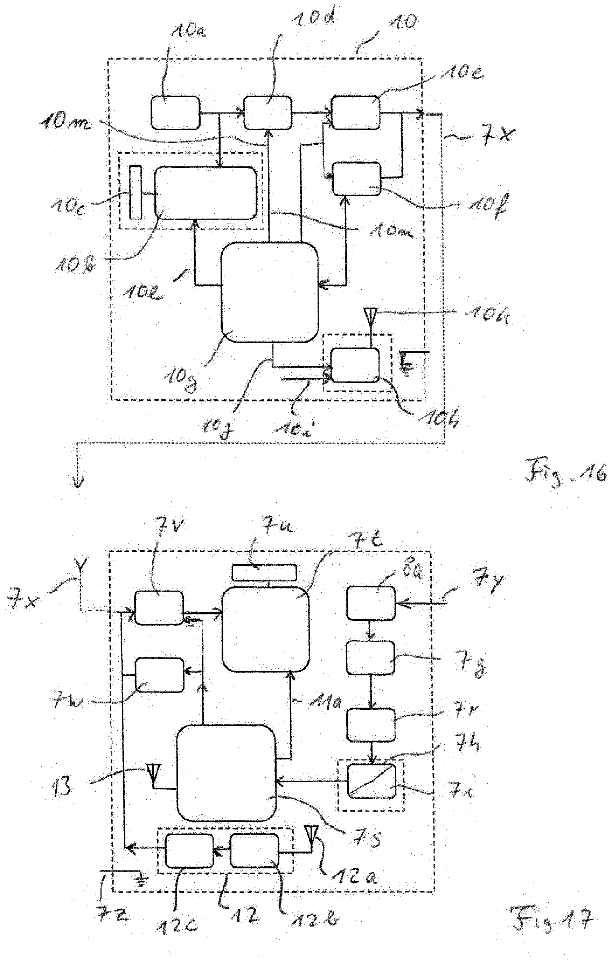

Figure 16 shows a detailed view of a charging station 10 for a mobile measuring unit of a temperature measurement device. In the figure, both possibilities are represented, a conducting contact between the charging station and the mobile measuring unit as well as a charging through a wireless interface like inductive charging which is indicated by an antenna 10k in figure 16. In reality, it is possible to realize in a charging station both or only one of the possibilities.

The charging station 10 comprises a USB connection 10b for power supply. From the USB connection, electric power is provided to a backup battery charger 10b which is connected to a backup battery 10c and charges the backup battery for emergency cases and the case of failure of the external power supply.

The USB connection is also connected to a voltage converter 10d which controls the voltage level of the voltage which is supplied to the mobile measuring unit. The voltage converter 10d is controlled by a pulse width modulation 10m which is provided by the microcontroller 10g. The microcontroller 10g also controls the backup battery charger 10b by a signal connection 10 l which makes use of a standardized communication protocol.

The microcontroller 10g also controls the two load switches 10e and 10f which both can establish or block the connection 7x to the mobile measuring unit 7, wherein load switch 10e controls the connection depending on the charging status while switch 10f has the function to protect the mobile measuring units in case of a short circuit or other kinds of failure.

The power line 7x establishes the electrically conductive power supply connection to the mobile measuring unit 7 in the case of charging. This hardware connection can for example be realized by metallic pressure contacts which may be spring loaded.

Figure 16 also shows an inductive field generator 10h which is controlled through a connection 10j by the microcontroller 10g, and which is connected to a power supply 10i. An antenna 10k is provided which can be realized by an inductive loop and which can couple with a respective loop in the mobile measuring unit in case of charging.

Figure 17 is showing a schematic view of a mobile measuring unit 7. The power supply of the unit 7 is provided by a rechargeable battery 7u. The battery is electrically connected to all elements of the unit 7 that need electric power supply, even if in the figure, the power supply connections are not shown for the sake of transparency of the drawing. The battery 7u is charged by a battery charger 7t which has the capacity to balance to a certain extent variable input voltages. The battery charger 7t is controlled by the microcontroller 7s through a signal line 11a which makes use of a standard protocol for the transfer of information.

The power supply of the battery charger 7t in case of charging can be provided through the power line 7x and alternatively through the inductive charging unit 12.

The power line 7x is connected to two load switches 7v, 7w of the mobile measuring unit, which can block or establish the electrical power supply connection between the power line 7x and the battery charger 7t.

The load switch 7v has the function to control the power supply connection from the functional perspective dependent on the charging status of the battery while load switch 7w can block the connection for protection reasons in case of failure.

In case the inductive charging unit 12 is used, the inductive loop 12a, which is symbolized as an antenna, is coupled with a respective loop 10k on the side of the charging station 10. A transformer 12b provides an ac power which is converted to a direct current and stabilized in the dc converter 12c. The dc converter 12 c is connected to the power line 7x and can supply power to the unit 7 in the same way as the hardware connection.

The mobile measuring unit on the measuring side comprises a pressure converter 8a, which can be a piezo converter, an interferometric converter or a pressure converter of any other kind mentioned in this text. The pressure converter 8a receives pressure waves through a pressure wave transfer element which might be a pressure wave transfer body 8 as shown in 13a or in figures 18ff. The transfer of the pressure waves in figure 17 is symbolized by the reference number 7y.

The pressure converter is converting transient pressure wave signals into electrical signals. These signals are supplied to a filter 7g which can be a high pass filter, a low pass filter or a band pass filter. The filtered signal is then supplied to an amplifier 7r which may e.g., have an amplification factor of 40-60dB.

The amplified signals are supplied to a signal integrator 7h and further to a signal averaging unit 7i, where the average over several signals can be calculated. The calculated values can then be sent to the microcontroller 7s and further sent via a wireless connection using the antenna 13, to a stationary receiving unit which can display a tissue temperature and send temperature values or control values to the controller of a therapy laser.

Between the microcontroller 7s and the analog switch 7w a connection line is established which is arranged to transmit signals according to a communication protocol as for example UART TX allowing for a comfortable, potentially bidirectional exchange of information.

As described above, in many cases for the detection and measuring of pressure waves, a ring-shaped piezo sensor is used. Such a ring is completely or at least partially made of a material with piezo effect, i.e., it develops an electric voltage signal as soon as a pressure on the material is applied or changed.

One advantage of piezo pressure converters is that they react very fast on pressure changes and hence can convert pressure changes of very high frequency.

One example of a ring-shaped piezo pressure converter or pressure transducer is shown in figure 18 in a perspective view. The ring 11 of figure 18 has a circular shape and a rectangular cross section. It has a first front side 11a which is also called the top side and which in use faces the eye of a patient, a second front side 11b which is also called the bottom side, an inner circumferential side (inner rim) 11d and an outer circumferential side (outer rim) 11c. The inner diameter of the ring is referenced with the reference sign 11e. The arrow 11f points into the direction of the eye of the patient, when the pressure converter is in use and fixed with its bottom side 11b to a pressure transfer body.

The thickness of the ring 11 is measured in its axial direction which is represented by the arrow 11g.

In figure 19, the ring 11 of figure 18 is shown in connection with a hollow cylindrical pressure wave transfer body 8. The eye of the patient has the reference sign 5. The pressure wave transfer body has a ring shaped opening or groove 8b with a rectangular cross section running around its inner circumference. The ring 11 is positioned in the opening 8b and fixedly connected to the bottom 8c of the opening 8b which is farther away from the eye. The ring can, e.g., be fixed with its second front side 11b (the bottom side) to the material of the pressure wave transfer body by an adhesive. The other three sides of the ring are not connected to the pressure wave transfer body. The outer circumferential side 11c as well as the first front side 11a of the ring have a distance to the material of the body 8. This distance can be left empty or filled by a decoupling, soft substance like a foam.

A pressure wave which is generated by an opto-acoustic pulse at the fundus of the eye 5 will travel through the eye and through the disposable contact element 7l and into the pressure wave transfer body 8. The pressure wave transfer body transfers the pressure waves to the bottom side 11b of the pressure converter 11 and the pressure converter vibrates and converts the vibration into electric signals.

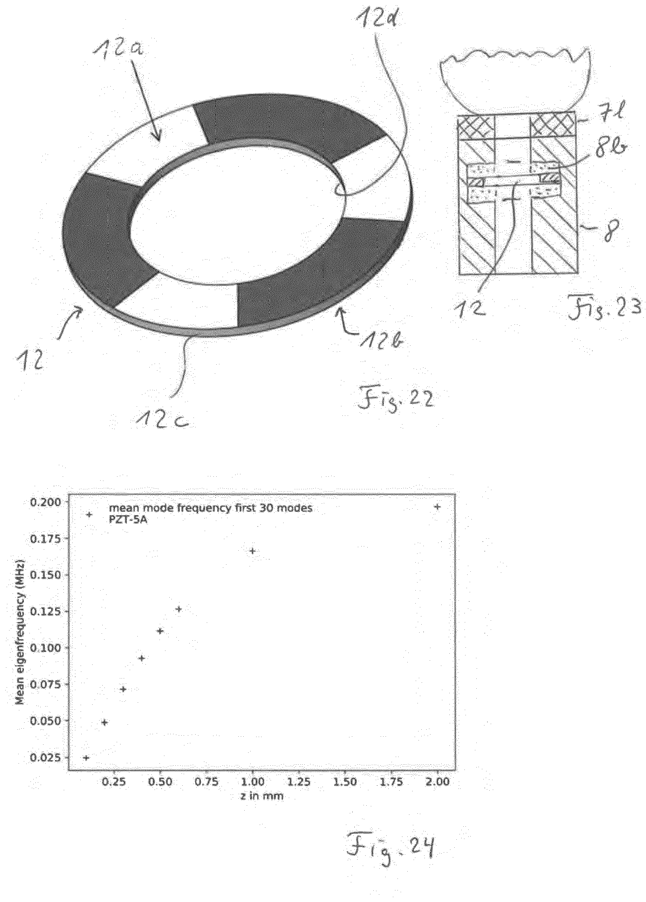

Figure 20 shows in a diagram the relation between the mean eigenfrequency of the ring 11 and the inner diameter of the ring which is shown in figure 18 for the case that its bottom side 11b is connected to the pressure wave transfer body 8 according to fig.19. As also for the diagrams of figures 21 and 24, the mean eigenfrequency has been calculated as the mean mode frequency of the first 30 vibrational modes of the ring 11.

It can be seen that the relation between the mean eigenfrequency and the inner diameter of the ring is clear, monotone and there is not a big change of the eigenfrequency if the inner diameter of the ring is changed between 10 and 15 mm. It is evident how the eigenfrequency of the ring can be changed by varying the inner diameter of the ring as long as the ring is fixed to the pressure wave transfer body 8 with the one of its front sides which during use is facing away from the patient's eye.

Figure 21 shows the relation between the mean eigenfrequency of the ring and the thickness of the ring as shown in figure 18 measured in its axial direction in case its bottom side of the ring facing away from the patient's eye is connected to a pressure wave transfer body as shown in fig.19.

It can be seen from figure 21 that the relation between the mean eigenfrequency of the ring 11 and the thickness of the ring is also transparent and clear. The mean eigenfrequency of the ring can be changed in a relatively wide range by varying the thickness of the ring between 0,1 mm and 0,5 mm. Would the ring be connected to the pressure wave transfer body in another way, e.g. by a fixed mechanical connection of more than one of its sides, then the relations as shown in figs.20 and 21 would be more complex, maybe not monotone, difficult to predict and an alignment or relative tuning of laser characteristics and the geometry and size of the pressure converter ring to one another would become way more difficult.

Figure 22 shows a perspective view of a pressure converter in the form of a piezo ring 12, which shall be at its outer circumferential side 12c fixedly connected by an adhesive to a pressure wave transfer body 8. The piezo ring 12 further has a first front side 12a, a second front side 12b and an inner circumferential side 12d.

Figure 23 shows that the pressure wave transfer body 8 has, as already shown in figure 19, a ring-shaped opening or groove 8b with a rectangular cross section which is running in azimuthal direction around its inner circumference.

The outer rim or outer circumferential side 12b of the ring 12 is either clamped into the groove 8b or connected to the bottom of the groove 8b by an adhesive. The two font sides 12a and 12b of the ring 12 as well as the inner circumferential side 12d are not touching the pressure wave transfer body 8.

On the sides 12a, 12b, a distance is provided between the material of the ring and the pressure wave transfer body 8. The distance can be left open or be filled with a decoupling material like a foam or gel. This material can also be chosen to form a damping layer for the ring which can be used to fine-tune the mean eigenfrequency.

In figure 23, the snap-in connection between the pressure wave transfer body 8 and the disposable contact element 7I is not shown but the connection my have a form analog to the connection shown in figure 13a or it can work with any other form of snap-in connection.

Figure 24 shows the relation between the mean eigenfrequency of the ring 12 and the thickness of the ring as shown in figure 18 in its axial direction in case the rim/outer circumference side of the ring 12 is connected to a pressure wave transfer body 8 as is shown in figure 23. It can be seen that there is a clear and monotone relation between the thickness z of the ring, measured in its axial direction, and the mean eigenfrequency. The variation of the thickness of the ring can be used in a predictable, transparent way to tune the mean eigenfrequency of the ring to characteristics of an excitation laser which is delivering the pulses for the temperature measurement or the measurement or determination of a control value.

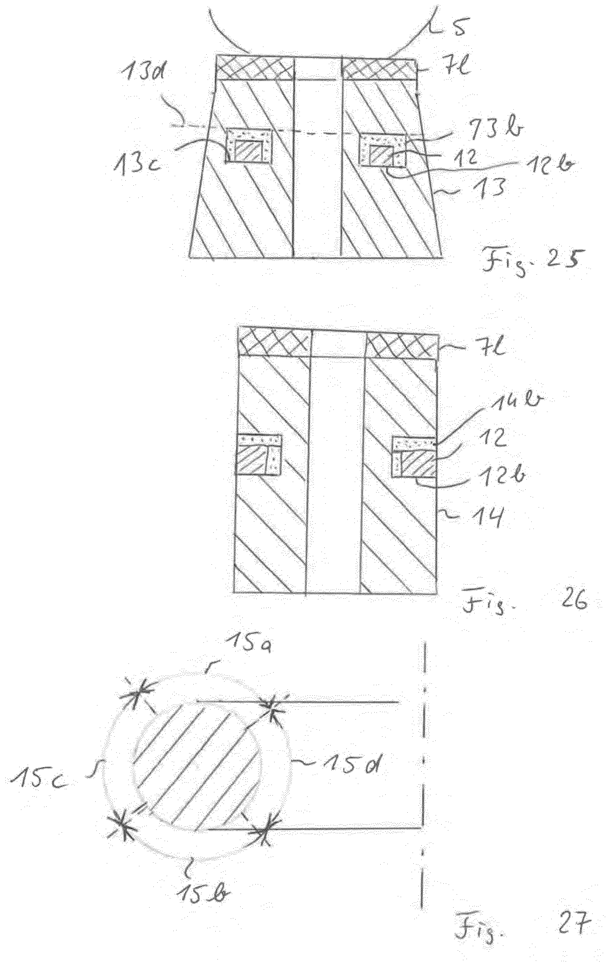

Figure 25 shows a cross sectional view of a pressure wave transfer body 13 of conical outer shape with a central cylindrical bore. A ring-shaped hollow space 13b of rectangular cross section is provided inside the pressure wave transfer body and in the hollow space, a pressure converter ring is positioned. The bottom side (second front side) 12b of the pressure converter is fixedly connected to the bottom 13 c of the hollow space, i.e., with the pressure wave transfer body. Each of the other sides of the pressure converter 12 is facing the material of the pressure wave transfer body with a distance which can be filled by a foam. The foam can protect the pressure converter from undesired influences. The pressure wave transfer body may be separable along the dotted line 13d and may consist of two parts which can be taken apart for introducing the pressure converter 12.

At the front end of the pressure wave transfer body, a disposable contact element 7I is provided which makes contact to the eye 5 of a patient.

Figure 26 shows a cross sectional view of a hollow cylindrical pressure wave transfer body 14 with an opening or groove 14b running in azimuthal direction around its outer circumferential side. The groove has a rectangular cross section and a pressure converter ring 12 is positioned in the groove. A bottom side (second front side) 12b of the pressure converter is fixedly connected to the pressure wave transfer body 14. The inner rim/inner circumferential side 12d of the pressure converter as well as its first front side have a distance to the material of the pressure wave transfer body. The distance may be filled with a foam in order to protect the pressure converter from environmental influences.

The pressure converter 12 has electrodes for deducting the voltage signal which is generated by the pressure changes. The electrodes are not shown in the figures and the setup is well known.

Figure 27 shows a cross sectional view of a pressure converter in the form of a toroid 15, i.e., a ring with a circular cross section. In this case of a ring of a not rectangular but circular cross section, the first front side 15a and the second front side 15bc, the inner circumferential side 15d and the outer circumferential side 15c of the ring 15 may be defined each as a surface section of 20-30% or an angular section of about 90 degrees of the circumference of the ring in the cross-sectional view as shown in the figure 27.

## Claims

1. Measuring device for measuring a control value, in particular the temperature, of the tissue of an eye (5) of a patient with an excitation source for pulse-like excitation of the tissue by means of a beam falling through the eye lens, in particular a laser beam (1b, 2b) or laser signal, with a measuring unit for placing on the patient's eye for the detection and/or measurement of pressure waves which are generated by opto-acoustic pulses (16a, 16b) in the tissue and transferred from the eye through a pressure wave transfer body (8, 13, 14) of the measuring unit to a pressure converter (7a, 8a, 11, 12) of the measuring unit for measuring transient pressure signals, wherein the pressure converter has the shape of a ring which at least partially consists of a piezoelectric material with an outer circumferential side (11c, 12c), an inner circumferential side (11d, 12d), a first front side (11a, 12a) and a second front side (11b, 12b) and is mechanically fixedly connected to the pressure wave transfer body, **characterized in that** only one of the sides of the ring, the outer circumferential side or the inner circumferential side or the first front side or the second front side is mechanically fixedly connected to the pressure wave transfer body, while the other sides are not mechanically coupled to the pressure wave transfer body.

2. Measuring device according to claim 1, **characterized in that** at least one, at least two or three of the sides (11a, 11b, 11c, 11d, 12a, 12b, 12c, 12d) of the ring-shaped pressure converter (7a, 8a, 11, 12) which is/are not mechanically coupled to the pressure wave transfer body (8, 13, 14), is/are facing a surface of the pressure wave transfer body directly or through a mechanical decoupling layer.

3. Measuring device according to claim 1 or 2, **characterized in that** the ring-shaped pressure converter (7a, 8a, 11, 12) is on at least three of its sides or completely surrounded by the material of the pressure wave transfer body (8, 13, 14).

4. Measuring device according to claim 1, 2, or 3, **characterized in that** the ring-shaped pressure converter (7a, 8a, 11, 12) is rounded, in particular oval or circular.

5. Measuring device according to claim 1, 2, 3 or 4 **characterized in that** the ring-shaped pressure converter (7a, 8a, 11, 12) has a rectangular cross section.

6. Measuring device according to one of claims 1 to 4, **characterized in that** the ring-shaped pressure converter (7a, 8a, 11, 12) has an oval or circular cross section.

7. Measuring device according to one of claims 1 to 6, **characterized in that** the ring-shaped pressure converter (7a, 8a, 11, 12) has at least one damping element which is damping the vibration of the ring.

8. Measuring device according to claim 7, **characterized in that** the ring-shaped pressure converter (7a, 8a, 11, 12) has an outer layer at least on one of its sides made of a material which is damping the vibration of the ring.

9. Measuring device according to one of claims 1 to 8, **characterized in that** the pressure wave transfer body (8, 13, 14) has the shape of a hollow cylinder or a hollow body which is cylindrical symmetric and/or that the pressure wave transfer body at a front end which is in use facing the eye of the patient, is provided with a disposable contact element for establishing direct contact with the eye of the patient, which is separably connected to the pressure wave transfer body by a snap-in connection and/or **characterized by** a receiving unit for the reception and evaluation of electrical signals which are generated by the pressure waves in the ring-shaped pressure converter (7a, 8a, 11, 12).

10. Method for operating a measuring device according to one of claims 1 to 9, **characterized by**:
aligning the pulse-like excitation signals of the excitation source and the shape and size of the ring-shaped pressure converter (7a, 8a, 11, 12) and its connection to the pressure wave transfer body in such a way that the distribution of the frequencies of the optoacoustic pressure waves has an initial shift relative to the distribution they would have to achieve the optimized resonance with the ring-shaped pressure converter (7a, 8a, 11, 12) or with the calculated mean mode frequency of the first 30 vibration modes of the ring-shaped pressure converter.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 13a

Fig. 13b

Fig. 15

10a    10d    10

10m

10e

~ 7x

10c

10b

10l

10m

10f

10g

10h

10g    10i

10h

Fig. 16

7v    7u    7t

Y

8a    7y

7x

7w

7g

13

~ 11a

7r

7h

7i

7s

12a

7z    12c    12    12b

Fig 17

Fig 18

Fig 19

Fig. 20

Fig 21

12a

12a

12

12b

12c

Fig. 22

7l

8b

8

12

Fig. 23

mean mode frequency first 30 modes
PZT-5A

Mean eigenfrequency (MHz)

0.200

0.175

0.150

0.125

0.100

0.075

0.050

0.025

0.25    0.50    0.75    1.00    1.25    1.50    1.75    2.00
z in mm

Fig. 24

13d
13c
5
7l
73b
12
12b
13

Fig. 25

7l
14b
12
12b
14

Fig. 26

15a
15c
15d
15b

Fig. 27

Europäisches
Patentamt

European
Patent Office

Office européen
des brevets

# EUROPEAN SEARCH REPORT

Application Number

EP 22 21 3247

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 279 385 A1 (MED LASERZENTRUM LUEBECK GMBH [DE]) 29 January 2003 (2003-01-29) | 1,2,4-9 | INV. A61F9/008 A61F9/009 |
| Y | * figure 3 * * paragraphs [0023] - [0027] * * figures 7,9 * | 3 | |
| Y | DE 199 32 477 A1 (MED LASERZENTRUM LUEBECK GMBH [DE]) 8 February 2001 (2001-02-08) * figures 1,2 * * paragraphs [0019] - [0026] * * figures 7-9 * | 3 | |
| A | DE 10 2012 009144 A1 (ZEISS CARL MEDITEC AG [DE]) 14 November 2013 (2013-11-14) * the whole document * | 1-10 | |

TECHNICAL FIELDS
SEARCHED      (IPC)

A61F
A61B

The present search report has been drawn up for all claims

| Place of search | Date of completion of the search | Examiner |
|---|---|---|
| Munich | 4 May 2023 | Jansen, Birte |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................................
& : member of the same patent family, corresponding document

**EP 4 385 468 A1**

## ANNEX TO THE EUROPEAN SEARCH REPORT
## ON EUROPEAN PATENT APPLICATION NO.

EP 22 21 3247

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2023

| Patent document cited in search report | | Publication date | Patent family member(s) | | Publication date |
|---|---|---|---|---|---|
| EP 1279385 | A1 | 29-01-2003 | DE | 10135944 A1 | 20-02-2003 |
| | | | EP | 1279385 A1 | 29-01-2003 |
| | | | US | 2003032949 A1 | 13-02-2003 |
| DE 19932477 | A1 | 08-02-2001 | DE | 19932477 A1 | 08-02-2001 |
| | | | US | 6671043 B1 | 30-12-2003 |
| DE 102012009144 | A1 | 14-11-2013 | DE | 102012009144 A1 | 14-11-2013 |
| | | | US | 2015157212 A1 | 11-06-2015 |
| | | | WO | 2013167274 A1 | 14-11-2013 |

EPO FORM P0459

For more details about this annex : see Official Journal of the European Patent Office, No. 12/82

**EP 4 385 468 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10135944 C2

- US 20030032949 A1